# EUROPEAN PATENT SPECIFICATION

(11) **EP 1 752 164 B1**
(45) Date of publication and mention of the grant of the patent: **07.05.2014**
(21) Application number: 05739196.3
(22) Date of filing: 13.05.2005
(51) Int. Cl.: A61K 47/08, A61K 9/70, A61K 31/192, A61K 45/08, A61K 47/14, A61K 47/16

(54) **PERCUTANEOUS PHARMACEUTICAL PREPARATION FOR EXTERNAL USE CONTAINING NONSTEROIDAL ANTIINFLAMMATORY ANALGESIC**
PERKUTANE PHARMAZEUTISCHE ZUBEREITUNG ZUR EXTERNEN ANWENDUNG MIT EINEM NICHSTEROIDALEN ENTZÜNDUNGSHEMMENDEN ANALGETIKUM
PRÉPARATION PHARMACEUTIQUE PERCUTANÉE POUR USAGE EXTERNE CONTENANT UN ANALGÉSIQUE ANTI-INFLAMMATOIRE NON STÉROÏDIEN

(30) Priority: 13.05.2004 JP 2004143945
(43) Date of publication of application: 14.02.2007
(73) Proprietor: HISAMITSU PHARMACEUTICAL CO., INC., Tosu-shi, Saga 841-0017 (JP)
(72) Inventor: HASHIMOTO, Yoshiaki, Tosu-shi, Saga 841-0017 (JP); TAKADA, Yasunori, Tosu-shi, Saga 841-0017 (JP); SHINMURA, Miyuki, Tosu-shi, Saga 841-0017 (JP); TSURUDA, Kiyomi, Tosu-shi, Saga 841-0017 (JP)
(74) Representative: Ehnis, Tobias
(86) International application number: PCT/JP2005/008748
(87) International publication number: WO 2005/110482

(56) References cited:
- WO-A1-01/68061
- JP-A- 7 126 121
- JP-A- 9 291 019
- JP-A- 60 155 111
- JP-A- 60 155 111
- JP-A- 61 215 315
- JP-A- 61 215 315
- JP-A- 61 215 316
- JP-A- 2000 136 122
- JP-A- 2002 284 622
- US-A- 5 637 293
- US-A- 5 709 847

## Description

### [Technical Field]

The invention relates to a percutaneous pharmaceutical preparation for external use containing a nonsteroidal antiinflammatory analgesic and, in particular, a percutaneous pharmaceutical preparation for external use containing a UV absorber to remarkably inhibit the photosensitivity, which appears as side effects of the nonsteroidal antiinflammatory analgesic.

### [background Art]

Since nonsteroidal antiinflammatory analgesics such as ketoprofen have an excellent antiinflammatory and analgesic actions, they are contained as an efficacious ingredient in each type of percutaneous pharmaceutical preparations for external use such as patches such as cataplasms and plasters, gels, creams, ointments and liniments. However, in a percutaneous pharmaceutical preparation for external use containing a nonsteroidal antiinflammatory analgesic, it is reported that the photosensitivity occurs infrequently as side effects (ref. none-patent document 1, patent document 3). It is also reported that photodecomposition generates byproducts which affect stability of a preparation, a sense of use and the like (ref. patent document 1).

As an approach to inhibit influence of light on nonsteroidal antiinflammatory analgesics, an example to try inhibition of photodecomposition products by preventing photodecomposition of ketoprofen while blending a UV absorber consisting of a benzophenone derivative to a preparation for external use containing ketoprofen (ref. patent document 1), an example to apply a UV blocking treatment to a backing of a patch containing a nonsteroidal antiinflammatory analgesic (ref. patent document 2), and an example to blend titanium oxide to a preparation for external use to the skin for inflammation (ref. patent document 3) have been reported.

On the other hand, although dibenzoylmethane derivatives are known as a UV absorber, compatibility with a base of a preparation for external use to the skin is poor, and therefore, it has been difficult to provide a stable preparation containing the dibenzoylmethane derivatives. In order to solve such a problem, blending a liquid ester of polyol fatty acid (ref. patent document 4), ester between acid with a specific group and alcohol (ref. patent document 5) or a specific diester (ref. patent document 6) has been tried. In addition, by using a UV protector together with a metal chelate agent, an art to sufficiently produce the effect of the UV protector without deterioration of blended ingredients (ref. patent document 7) and the like have been proposed.

However, as to the above approaches to inhibit the influence of light on a nonsteroidal antiinflammatory analgesic, it could not be said that any approach completely inhibited a side effect to the skin. In the meantime, a preparation for external use to the skin blended with the benzoylmethane derivative, the above ester, and the like still has problems in an aging stability of a blended ingredient and in efficacy, and therefore, a further improvement of preparation has been desired.
Patent document 1: JP, A, 60-155111
Patent document 2: WO 01/68061
Patent document 3: JP, A, 9-169658
Patent document 4: JP, A, 61-215315
Patent document 5: JP, A, 61-215316
Patent document 6: JP, A, 9-291019
Patent document 1: JP, A, 2000-136122
None-patent document 1: New Pharmacology, 3rd revised edition, Nankodo, 25, 10, 1996, pp.474-476, edited by Chikako Tanaka and Ryuichi Kato

### [Disclosure of the Invention]

### [Problems to Be Solved by the Invention]

Consequently, the object of the invention is to inhibit, with higher certainty, side effects to the skin of a percutaneous pharmaceutical preparation for external use containing a nonsteroidal antiinflammatory analgesic as well as to prevent the preparation from alteration with time, to provide the percutaneous pharmaceutical preparation for external use which sufficiently produce substantial effects of the nonsteroidal antiinflammatory analgesic.

### [Means to Solve the Problems]

As to a drug photosensitivity, during extensive research to solve the above problems, the inventors noted that since there are phototoxicity occurring due to a non-immunological mechanism in case that a drug is exposed to sunlight and photoallergy in which a haptenic drug generated by exposure of sunlight brings a damage to tissues/cells via an immunological mechanism; the former is indifferent to predisposition of an individual and anyone has a possibility of the onset if exposed with plenty UV rays, and the latter occurs in only some individuals sensitized with a haptenic drug in an allergic way and symptoms occurs independently of a dose of the drug or sunlight exposure level, it is necessary to inhibit these both mechanisms in order to prevent the photosensitivity certainly.

In addition, the inventors found that, in particular, by blending a dibenzoylmethane derivative as a UVA absorber, which particularly absorbs ultraviolet-A (UVA: wave length 320-400nm), in a preparation at a high concentration, both of the above phototoxicity and photoallergy due to a nonsteroidal antiinflammatory analgesic could remarkably be inhibited and the effects which the nonsteroidal antiinflammatory analgesic essentially had, could sufficiently be produced, together with improving compatibility of the dibenzoylmethane derivative in the preparation by blend of a specific resolvent, and the inventors accomplished the percutaneous pharmaceutical preparation for external use of the invention.

Namely, the invention relates to a percutaneous pharmaceutical preparation for external use comprising, as essential ingredients, a nonsteroidal antiinflammatory analgesic, a dibenzoylmethane derivative, and one or more selected from polyol fatty acid esters, higher fatty acid esters, and crotamiton.

The nonsteroidal antiinflammatory analgesic is one selected from the group consisting of ketoprofen and pharmaceutically acceptable salts thereof.

The dibenzoylmethane derivative is 4-tert-butyl-4'-methoxydibenzoylmethane.

Further, the invention relates to the percutaneous pharmaceutical preparation for external use, wherein the content of the dibenzoylmethane derivative is 0. 1 to 20 weight %.

The invention also relates to the percutaneous pharmaceutical preparation for external use, wherein the polyol fatty acid ester is one or more selected from the group consisting of propylene glycol monocaprylate, propylene glycol dicaprylate, caprylic/capric triglyceride and glyceryl tri-2-ethylhexanoate.

The invention furthermore relates to the percutaneous pharmaceutical preparation for external use, wherein the higher fatty acid ester is one or more selected from the group consisting of isopropyl myristate, isopropyl palmitate, cetyl isooctanoate, diethyl sebacate, diisopropyl adipate and dioctyl adipate.

In addition, the invention relates to the percutaneous pharmaceutical preparation for external use, wherein the higher fatty acid ester is one or more selected from the group consisting of higher fatty acid esters which are liquid at ordinary temperatures.

The invention relates to the percutaneous pharmaceutical preparation for external use, wherein the content of the nonsteroidal antiinflammatory analgesic is 0.1 to 10 weight %, the content of the dibenzoylmethane derivative is 0.1 to 20 weight %, and the content of one or more selected among the polyol fatty acid esters, the higher fatty acid esters, and crotamiton is 0.1 to 10 weight %.

The invention also relates to the percutaneous pharmaceutical preparation for external use, wherein the nonsteroidal antiinflammatory analgesic is ketoprofen, the dibenzoylmethane derivative is 4-*tert*-butyl-4'-methoxy-dibenzoylmethane, and one or more selected among the polyol fatty acid esters, the higher fatty acid esters, and crotamiton are one or more selected among diisopropyl adipate, propylene glycol dicaprylate, caprylic/capric triglyceride, glyceryl tri-2-ethylhexanoate, and crotamiton.

The invention further relates to the percutaneous pharmaceutical preparation for external use, wherein the dosage form of the percutaneous pharmaceutical preparation for external use is a cataplasm or a plaster.

### [Effect of the invention]

According to the percutaneous pharmaceutical preparation for external use of the invention, the photosensitivity caused by a nonsteroidal antiinflammatory analgesic can be remarkably inhibited by containing a dibenzoylmethane derivative as a UV absorber at a high concentration. Namely, according to the percutaneous pharmaceutical preparation for external use of the invention, both of the phototoxicity and photoallergy caused by the nonsteroidal antiinflammatory analgesic can be remarkably inhibited by producing a UV absorption effect depending on a blend amount of the dibenzoylmethane derivative.

In addition, by blending one or more selected among polyol fatty acid esters, higher fatty acid esters, and crotamiton as a resolvent, a percutaneous pharmaceutical preparation for external use of the invention can very stably be blended with a dibenzoylmethane derivative of a high concentration in a preparation, which was conventionally difficult in making a stable preparation due to its poor compatibility with other bases.

Further, even if a percutaneous pharmaceutical preparation for external use of the invention is blended with a dibenzoylmethane derivative and a resolvent such as the above esters and crotamiton, without reducing permeability of a nonsteroidal anti-inflammatory analgesic which is an efficacious ingredient to the skin from a preparation, it sufficiently produces effects which the nonsteroidal antiinflammatory analgesic essentially has.

That is, the percutaneous pharmaceutical preparation for external use of the invention inhibits, with higher certainty, the photosensitivity caused by the phototoxicity and photoallergy of a nonsteroidal antiinflammatory analgesic, and ingredients blended in the preparation can stably exist without alteration with time and sufficiently exhibit an antiinflammatory analgesic effect of the nonsteroidal antiinflammatory analgesic; thus, the percutaneous pharmaceutical preparation for external use containing the nonsteroidal anti-inflammatory analgesic and having such effects was realized for the first time by the invention.

### [Best Embodiment for carrying out the Invention]

In the following, embodiments of the percutaneous pharmaceutical preparation for external use of the invention are illustrated in detail.

The nonsteroidal antiinflammatory analgesic which can be used in the percutaneous pharmaceutical preparation for external use of the invention is ketoprofen or a pharmaceutically acceptable salt thereof; ketoprofen has a benzophenone backbone.

The blend amount of the above nonsteroidal antiinflammatory analgesic in the percutaneous pharmaceutical preparation for external use of the invention is not particularly limited; however, it is preferably 0.1 to 10 weight % based on the total amount of the preparation, more preferably 0.5 to 5 weight %, furthermore preferably 1 to 3 weight %.

The dibenzoylmethane derivative used in the percutaneous pharmaceutical preparation for external use of the invention is 4-*tert*-butyl-4'-methoxydibenzoylmethane. 4-*tert*-Butyl-4'-methoxydibenzoylmethane is an excellent UVA absorber which has the maximum absorption in about 330-360nm, and PARSOL 1789 (manufactured by Roche Co., Ltd.) and the like can be used as a commercial product.

The blend amount of the dibenzoylmethane derivative in the percutaneous pharmaceutical preparation for external use of the invention is not particularly limited; however, in order to let it produce an inhibitory effect against the phototoxicity and photoallergy caused by a nonsteroidal antiinflammatory analgesic, it is preferably 0.1 to 20 weight % based on the total amount of the preparation, more preferably 1 to 10 weight %, furthermore preferably 3 to 6 weight %.

In the invention, one or more selected from polyol fatty acid esters, higher fatty acid esters, and crotamiton can be used as a resolvent. The above resolvent used in the invention is preferably a solvent that is good in compatibility for a nonsteroidal antiinflammatory analgesic which is an efficacious ingredient. In addition, in case that the nonsteroidal antiinflammatory analgesic used in the invention has a carboxyl group, the above resolvent is preferably a compound having no hydroxyl group in order not to form an ester between the above resolvent and the nonsteroidal anti-inflammatory analgesic. Further, as the polyol fatty acid ester used in the invention, an ester between a polyol and a C₈-C₁₀ fatty acid having a straight chain or branched chain alkyl groups is preferable. Furthermore, as the higher fatty acid ester used in the invention, an ester between a C₆-C₁₉ fatty acid having a straight chain or branched chain alkyl groups and a C₂-C₁₈ alcohol having a straight chain, branched chain or unsaturated alkyl groups, and/or a higher fatty acid diester which is liquid at ordinary temperatures is preferable.

Although the polyol fatty acid ester used in the percutaneous pharmaceutical preparation for external use of the invention is not particularly limited as long as it is liquid at ordinary temperatures, an ester between a polyol and a C₈-C₁₀ fatty acid having a straight chain or branched chain alkyl groups is preferable. Examples of the polyol fatty acid esters used in the invention include propylene glycol monocaprylate, propylene glycol dicaprylate, propylene glycol monocaprate, propylene glycol dicaprate, caprylic/capric triglyceride, glyceryl tri-2-ethylhexanoate, 2-butyl-2-ethyl-1,3-propanediol 2-ethylhexanoate, etc. Propylene glycol dicaprylate, caprylic/capric triglyceride and glyceryl tri-2-ethylhexanoate are particularly preferable polyol fatty acid esters.

The higher fatty acid ester used in the percutaneous pharmaceutical preparation for external use of the invention is not particularly limited as long as it is an ester between a C₆-C₁₉ fatty acid having a straight chain or branched chain alkyl groups and a C₂- C₁₈ alcohol having a straight chain, branched chain or unsaturated alkyl groups, and/or a higher fatty acid diester which is liquid at ordinary temperatures. Examples of the esters between a C₆- C₁₉ fatty acid having a straight chain or branched chain alkyl groups and a C₂- C₁₈ alcohol having a straight chain, branched chain or unsaturated alkyl groups, which are used in the invention, include butyl myristate, isopropyl myristate, 2-hexyldecyl myristate, cetyl isoocanoate, isopropyl palmitate, 2-ethylhexyl palmitate, isosearyl palmitate, ethylhexyl stearate, 2-hexyldecyl stearate, isopropyl isostearate, 2-hexyldecyl isostearate, etc. Among these, isopropyl myristate, isopropyl palmitate and cetyl isoocanoate are particularly preferable.

Additionally, in the invention, a higher fatty acid diester which is liquid at ordinary temperatures may be an ester between a C₆-C₁₀ dibasic acid and a C₂-C₈ alcohol having a straight chain or branched chain alkyl groups. Examples include diethyl sebacate, diisopropyl sebacate, diisopropyl adipate and dioctyl adipate. Among these, diethyl sebacate and diisopropyl adipate are particularly preferable. Further, in the invention, ordinary temperatures mean 15-25°C.

Crotmiton in the percutaneous pharmaceutical preparation for external use of the invention is one of N-substituted-O-toluidine derivatives and excellent in solubility of a wide range of drugs from a fat-soluble drug to a hydrophilic drug, and therefore, it is favorably used in preparations such as patches including cataplasms, plasters, etc., ointments, creams, and the like.

In the percutaneous pharmaceutical preparation for external use of the invention, the blend amount of (C) one or more selected among the polyol fatty acid esters, the higher fatty acid esters, and crotamiton in the preparation is preferably 1/10 to 1 against the blend amount of (B) the dibenzoylmethane derivative. In addition, the blend amount of one or more selected among (C) the polyol fatty acid esters, the higher fatty acid esters, and crotamiton in the preparation is preferably 0.1 to 10 weight %, more preferably 0.5 to 5 weight %, furthermore preferably 1 to 3 weight %, based on the total amount of the preparation, from the viewpoint of an aging stability of the preparation and permeability of an efficacious ingredient to the skin.

Further, by containing (B) the dibenzoylmethane derivative and (C) one or more selected among the polyol fatty acid esters, the higher fatty acid esters, and crotamiton, (A) the nonsteroidal antiinflammatory analgesic does not deteriorate, whereby its permeability to the skin does not lower, and an essential effect of the nonsteroidal antiinflammatory analgesic is not inhibited.

In the invention, "inhibit" means that values on the phototoxicity and photoallergy evaluated by a phototoxicity test and a photoallergy test (ref. test examples 1 and 2) decrease respectively comparing with those in case of not containing the above dibenzoylmethane derivative, by containing the above dibenzoylmethane derivative. The degree of decrease (inhibition ratio) is preferably not less than 30%, more preferably not less than 40%, furthermore preferably not less than 50%, and most preferably not less than 60%.

In the percutaneous pharmaceutical preparation for external use of the invention, except the above essential ingredients other bases for each preparation may be blended depending on a dosage form of the preparation. In addition, as the dosage form of the percutaneous pharmaceutical preparation for external use of the invention illustrative are patches such as cataplasms or plasters, etc. In the following, the bases as well as the formula examples depending on the dosage form of the percutaneous pharmaceutical preparation for external use of the invention are illustrated.

### [Examples]

In the following, the invention is explained in more detail by examples; however, the present invention is not limited to these examples, and various modification is possible without departing from the technical idea of the invention.

As an example, a plaster is explained. A plaster base used in the plaster of the invention is not particularly limited and selected among those usually used. As ingredients contained in such a plaster base, examples include polymer bases (an acrylic composition which is copolymer with methacrylate, acryronitrile, or a vinyl monomer such as vinyl acetate or vinyl propionate, a silicon resin, a polyisoprene rubber, a natural rubber, an acrylic rubber, a styrene-butadiene-styrene block copolymer, a styrene-isoprene-styrene block copolymer, etc.), oils or higher fatty acids (almond oil, olive oil, camellia oil, persic oil, peanut oil, oleic oil, liquid paraffin, polybutene, etc.), tackifiers (rosin, a maleic acid-modified rosin ester, a hydrogenated rosin ester, etc.), fatty acid metal salts (zinc undecylenate, zinc stearate, calcium stearate, aluminum stearate, magnesium stearate, sodium stearate, zinc palmitate, zinc myristate, magnesium myristate, sodium laurate, zinc laurate, etc.), rash preventing agents and other additives (salicylic acid, methyl salicylate, glycol salicylate, 1-menthol, camphor, vanillyl amide nonylate, red pepper extract, peppermint oil, Azone (trade mark), etc.), and the like. The plasters of the invention can be obtained by blending the above essential ingredients in a plaster base which is prepared by mixing each ingredient selected among these.

Next, one favorable preparation example of plasters (formula example) is shown. Namely, in case of preparing by a hot-melt method, first, the above polymer base, the above oil or fatty acid, the above tackifier and the above fatty acid metal salt are heated and mixed at 120-160°C using a kneader, a mixer or the like, added with the above nonsteroidal antiinflammatory analgesic, the above dibenzoylmethane derivative, and one or more selected among the above polyol fatty acid esters, higher fatty acid esters, and crotamiton, and mixed. The resulting mixture may directly be spread over a backing, or once spread over paper, film, or the like in which a removable treatment is carried out, and then transferred to a desirable support in a covered state by compression. On the other hand, in case of preparing by a solvent method, the above ingredients are dissolved in solvent such as toluene, hexane, methylene chloride or the like using a mixing machine such as a mixer with anti-explosion treatment, or the like. The resulting solution is spread over paper, film, or the like in which a removable treatment is carried out, dried by a drying machine to remove solvent, and then transferred to a desirable support in a covered state by compression. A removable cover is stuck on the spread coat on the support to obtain the plasters of the invention. As such a support, specifically, examples includefabric, non-woven fabric, woven fabric, polyurethane, polyester, polyvinyl acetate, polyvinylidene chloride, polyethylene, polyethylene terephthalate, aluminum sheet, or the like, or a composite material thereof. In addition, as to the removable cover, examples include polyethylene, polypropylene, polyvinyl chloride, polyester, polyvinylidene chloride, paper treated with silicon, etc.

As described above, favorable embodiments of the bases and the formula examples depending on a dosage form of the percutaneous pharmaceutical preparation for external use of the invention were explained; however, the dosage forms as well as the formula examples are not limited to these, and a blending order of each ingredient is not particularly limited.

Additionally, in the percutaneous pharmaceutical preparation for external use of the invention, an antioxidant may further be blended in addition to the above formula. As such an antioxidant preferable are phenol derivatives,such as butyl hyroxyanisole, dibutyl hydroxytoluene, thymol and propyl gallate, tocopherol and its ester derivatives, ascorbic acid and its ester derivatives, etc. Such antioxidants may be used alone, or two or more may be used in combination. Although the blend amount is not particularly limited, preferably 0 to 10 weight %, and more preferably 0 to 5 weight %, based on the total amount of the preparation.

In the following, the invention is explained in more detail by test examples. Here, in the following examples, "%" means "weight %" unless otherwise specified.

### Test example 1: Phototoxicity test (Ear edema inhibition test using mice)

Styrene-isoprene-styrene block copolymer (SIS), polyisobutylene (PIB), hydrogenated rosin glycerol ester (KE-311), liquid paraffin and zinc stearate were weighed according to weight % in Table 1, heated and stirred under an atmosphere of nitrogen gas to obtain a solution (Step A). The stirring temperature was 100-200°C, and the stirring time was 30-120 minutes.

Subsequently, ketoprofen (KP), 1-menthol, diisopropyl adipate (DID) and a designated amount of 4-*tert*-butyl-4'-methoxydibenzoylmethane (BM-DBM) were added to the solution of the above step A in the range of 100-200 °C of temperature and mixed for 5-30 minutes to obtain a uniform solution (Step B). The solution obtained in the step B was spread over a silicone-treated polyethylene terephthalate (PET) film at a weight of 1 g per 70 cm², then covered with a polyester non-woven fabric, transferred by compression, and cut to a desired size to obtain the plasters of the invention (Examples 1-5).

**[Table 1]**

| Formula | (Example 1) | (Example 2) | (Example 3) | (Example 4) | (Example 5) |
|---|---|---|---|---|---|
| SIS | 29.5 | 29.5 | 29.5 | 29.5 | 29.5 |
| Liquid paraffin | 33.5 | 33.5 | 32.5 | 31.0 | 29.0 |
| PIB | 10 | 10 | 10 | 10 | 10 |
| KE-311 | 18.5 | 18.0 | 17.0 | 15.5 | 13.5 |
| Zinc stearate | 2 | 2 | 2 | 2 | 2 |
| KP | 2 | 2 | 2 | 2 | 2 |
| 1-Menthol | 3 | 3 | 3 | 3 | 3 |
| BM-DBM | 0.5 | 1 | 3 | 6 | 10 |
| Diisopropyl adipate | 1 | 1 | 1 | 1 | 1 |
| Total | 100 | 100 | 100 | 100 | 100 |

A test for an ear edema inhibition ratio in mice was carried out according to the method of Gerberic et al. (Food Chem. Toxicol., 27, 813-819 (1989)) using the ketoprofen preparations of the examples 1-5. That is, as a test animal, Balb/c mice (female, 9-11 week old) were used. The plasters (the examples 1-5) containing ketoprofen (KP) of 2% and 4-*tert*-butyl-4'-methoxydibenzoylmethane of 0.5%, 1%, 3%, 6% and 10% respectively were stuck on the concha for 4 hours, and then irradiated with UVA at 40J/cm². In addition, a group coated with 2% ethanolic solution of ketoprofen (KP) was made a control group.

An ear thickness after 24 hours of the UVA irradiation was measured, and an increasing portion from the ear thickness before the start of the test was calculated. An inhibition effect on the phototoxicity by ketoprofen in the preparation containing BM-DBM of each concentration was evaluated by the ear edema inhibition ratio (%) as an indicator that the increase can be inhibited in any degree against the increasing portion of the ear thickness in the control group. The obtained results are shown in Table 2.

**[Table 2]**

| BM-DBM (%) | Ear edema inhibition ratio (%) |
|---|---|
| 0.5 | 27 |
| 1 | 46 |
| 3 | 62 |
| 6 | 85 |
| 10 | 92 |

As the results of the test for the ear edema inhibition ratio using mice are shown in Table 2, in the ketoprofen containing preparations of the examples 1-5, which were blended with 4-*tert*-butyl-4'-methoxydibenzoylmethane (BM-DBM), the action to remarkably reduce the phototoxicity by ketoprofen depending on a dose as the BM-DBM content increased, was observed.

### Test example 2: Photoallergy test (Skin photoallergy test using guinea pigs)

Using the ketoprofen preparations of the examples 3-5, a skin photoallergy test using guinea pigs was carried out according to the method of Adjuvant and Strip method by Sato et al. (Nishinihon Hifuka, 42, 831-837 (1980)). That is, the cervical back of a white female Hartley strain guinea pig (one group: eight guinea pigs) was depilated, and the adjuvant was administered to four corners of 2×2cm, and then, 2% ketoprofen solution dissolved in ethanol was coated on the above parts of 2×2cm as the control group, or the plasters (the examples 3-5, 2×2cm) containing ketoprofen (KP) of 2% and 4-*tert*-butyl-4'-methoxydibenzoylmethane of 3%, 6% or 10% respectively were stuck for 4 hours. After coating the solution or one hour after peeling off of the plasters, UVA (10 J/cm²) was irradiated. This treatment of sensitization induction was carried out successively for 5 days. After 3 weeks from the start of the sensitization, the lumber back was depilated, and the same samples when sensitizing on the area of 2×2cm were applied, and then, after 1 hour, photo-induction was carried out irradiating UVA (10 J/cm²). Skin reactions after 24 hours and 48 hours from the irradiation were evaluated according to the above standard of Sato et al. The inhibition effect of the BM-DBM containing preparations (the examples 3-5) with different blend amounts were evaluated by the inhibition ratio (%) of erythema and edema against the control group coated with the ketoprofen solution. The obtained results are shown in Table 3.

**[Table 3]**

| BM-DBM concentration (%) | Erythema · Edema inhibition ratio (%) | |
|---|---|---|
| | After 24 hrs | After 48 hrs |
| 3 | 20 | 12 |
| 6 | 65 | 61 |
| 10 | 63 | 59 |

As shown in table 3 on the results of the skin photoallergy test using guinea pigs, it was observed that the ketoprofen containing preparations blended with 4-*tert*-butyl-4'-methoxydibenzoylmethane (BM-DBM) of the examples 3-5 reduced remarkably the photoallergy by ketoprofen as the BM-DBM content increased.

As is evident from the results shown in Tables 2 and 3, in the percutaneous pharmaceutical preparations for external use containing the nonsteroidal antiinflammatory analgesic as an efficacious ingredient, it is understood that the photoallergy and phototoxicity by the nonsteroidal antiinflammatory analgesic are remarkably inhibited by blend of the dibenzoylmethane derivative as a UVA absorber.

### Test example 3: Aging stability test of preparation

Styrene-isoprene-styrene block copolymer (SIS), polyisobutylene (PIB), hydrogenated rosin glycerol ester (KE-311), liquid paraffin and zinc stearate were weighed according to weight % in the following Table, and heated and stirred under an atmosphere of nitrogen gas to obtain a solution (Step A). The stirring temperature was 100-200°C, and the stirring time was 30-120 minutes.

Subsequently, ketoprofen (KP), 1-menthol, 4-*tert*-butyl-4'-methoxydibenzoylmethane (BM-DBM) and a designated amount of diisopropyl adipate (DID) were added to the solution of the above step A in the range of 100-200°C of temperature and mixed for 5-30 minutes to obtain a uniform solution (Step B). The solution obtained in the step B was spread over a silicone-treated polyethylene terephthalate (PET) film at a weight of 1 g per 70 cm², then covered with a polyester non-woven fabric, transferred by compression, and cut to a desired size to obtain the plasters of the invention (Examples 6 and 7). Further, the plasters in which the above diisopropyl adipate was changed to crotamiton 3%, propylene glycol dicaprylate 1%, caprylic/capric triglyceride 2%, or glyceryl tri-2-ethylhexanoate 2% were obtained (Examples 8-11). In addition, as comparative examples, the plasters in which the above diisopropyl adipate was changed to propylene carbonate 3% or triacetin 3% were prepared (Comparative Examples 1 and 2).

**[Table 4]**

| Formula (wt.%) | Ex.6 | Ex.7 | Ex.8 | Ex.9 | Ex.10 | Ex.11 | Comp.Ex.1 | Comp.Ex.2 |
|---|---|---|---|---|---|---|---|---|
| SIS | 29.5 | 29.5 | 29.5 | 29.5 | 29.5 | 29.5 | 29.5 | 29.5 |
| Liquid paraffin | 30.5 | 30 | 30 | 31 | 30.5 | 30.5 | 30 | 30 |
| PIB | 10 | 10 | 10 | 10 | 10 | 10 | 10 | 10 |
| KE-311 | 15 | 14.5 | 14.5 | 15.5 | 15 | 15 | 14.5 | 14.5 |
| Zinc stearate | 2 | 2 | 2 | 2 | 2 | 2 | 2 | 2 |
| KP | 2 | 2 | 2 | 2 | 2 | 2 | 2 | 2 |
| 1-Menthol | 3 | 3 | 3 | 3 | 3 | 3 | 3 | 3 |
| BM-DBM | 6 | 6 | 6 | 6 | 6 | 6 | 6 | 6 |
| Diisopropyl adipate | 2 | 3 | | | | | | |
| Crotamiton | | | 3 | | | | | |
| Propylene glycol dicaprylate | | | | 1 | | | | |
| Caprylic/capric triglyceride | | | | | 2 | | | |
| Glyceryl tri-2-ethylhexanoate | | | | | | 2 | | |
| Propylene carbonate | | | | | | | 3 | |
| Triacetin | | | | | | | | 3 |
| Total | 100 | 100 | 100 | 100 | 100 | 100 | 100 | 100 |

After storing each preparation (Examples 6-11, and Comparative Examples 1 and 2) at ordinary temperatures for a designated period, the presence or absence of crystal precipitation of 4-*tert*-butyl-4'-methoxydibenzoylmethane (BM-DBM), or the presence or absence of stickiness of a surface of the adhesive mass were observed, and the results are shown in Table 5.

As shown in the results of Table 5, in the preparation blended with propylene carbonate 3% (Comparative Example 1) and the preparation blended with triacetin 3% (Comparative Example 2), crystal precipitation was observed immediate after the production, whereby the cohesive strength was insufficient, the adhesive mass was rather soft, the preparation produced stringiness, and a tendency to feel stickiness on the surface of the adhesive mass was noted. On the contrary, in the ketoprofen preparations of Examples 6-11, the physical properties in each of the preparations are favorable, no crystal precipitation was observed after 2 or more months at ordinary temperatures, and no stickiness on the surface of the adhesive mass was also noted.
[Table 5]

**Table 5: Observation results of preparation**

| | Resolvent | Blend amount | Observation results of preparation after production | |
|---|---|---|---|---|
| | | | Presence and absence of crystal | Stickiness of adhesive mass |
| Ex.6 | Diisopropyl adipate | 2% | No | No |
| Ex.7 | | 3% | No | No |
| Ex.8 | Crotamiton | 3% | No | No |
| Ex.9 | Propylene glycol dicaprate | 1% | No | No |
| Ex.10 | Caprylic/capric triglyceride | 2% | No | No |
| Ex.11 | Glyceryl tri-2-ethylhexanoate | 2% | No | No |
| Comp.Ex.1 | Propylene carbonate | 3% | Yes | Yes |
| Comp.Ex.2 | Triacetin | 3% | Yes | Yes |

As is evident from the results shown in Table 5, it is understood that in the percutaneous pharmaceutical preparations for external use containing a nonsteroidal anti-inflammatory analgesic blended with a hardly-soluble dibenzoylmethane derivative, it is possible to make the preparations excellent in an aging stability by use of polyol fatty acid esters, higher fatty acid esters, and crotamiton as a resolvent.

### Test example 4: Skin permeability test (in vitro skin permeability test using hairless mice)

A back part skin of a hairless mouse was stripped, and its dermal side was placed to a receptor layer side and installed on a flow-through cell (0.785cm²) in which warm water of 37° C was circulated around the outer part. The preparations obtained in Examples 4 and 6-8 were stuck on the stratum corneum side, and sampling was carried out at every 4 hours for 24 hours at a rate of 0.8 ml/hr. As for the receptor side, saline was used. The amount of ketoprofen in the receptor solution obtained at each time was measured by high-performance liquid chromatography, and the ketoprofen skin permeation rate per hour from the preparation was calculated. The results are shown in Table 6.
[Table 6]

**Table: Results of in vitro skin permeability test in hairless mice**

| Resolvent | Blend amount | Skin permeation rate [µg/cm²/hr] | ± S.D. |
|---|---|---|---|
| Diisopropyl adipate | 1% | 14.2 | 0.8 |
| | 2% | 12.7 | 0.8 |
| | 3% | 11.4 | 1.3 |
| Crotamiton | 3% | 13.6 | 0.8 |

As shown in Table 6, even if the blend amount of diisopropyl adipate (DID) was increased, almost no reduction of the skin permeation rate of ketoprofen was confirmed. It is also demonstrated that the skin permeation rate was hardly reduced in case of blending crotamiton 3%.

Therefore, it is understood that the percutaneous pharmaceutical preparation for external use according to the invention does not reduce the skin permeability of a nonsteroidal antiinflammatory analgesic contained in the preparation and can sufficiently produce its efficacy even if a dibenzoylmethane derivative and a resolvent such as a polyol fatty acid ester, a higher fatty acid ester, or crotamiton are blended.

### [Industrial Applicability]

As explained above, according to the invention, in a percutaneous pharmaceutical preparation for external use containing a nonsteroidal antiinflammatory analgesic, it becomes possible to sufficiently produce the antiinflammatory analgesic effects while certainly preventing the photosensitivity caused by the phototoxicity and photoallergy, and therefore, application as a drug which is extremely high in an aging stability can be expected.

## Claims

1. A percutaneous pharmaceutical preparation for external use comprising the following ingredients (A), (B) and (C);
(A) a nonsteroidal antiinflammatory analgesic,
(B) a dibenzoylmethane derivative,
(C) one or more selected from polyol fatty acid esters, higher fatty acid esters, and crotamiton
wherein the dibenzylmethane derivate is 4-*tert*-butyl-4'-methoxydibenzoylmethane and the nonsteroidal anti-inflammatory analgesic is ketoprofen or a pharmaceutically acceptable salt thereof.

2. The percutaneous pharmaceutical preparation for external use according to claim 1, wherein the content of the dibenzoylmethane derivative is 0.1 to 20 weight %.

3. The percutaneous pharmaceutical preparation for external use according to any one of claims 1 or 2, wherein the polyol fatty acid ester is one or more selected from the group consisting of propylene glycol monocaprylate, propylene glycol dicaprylate, caprylic/capric triglyceride and glyceryl tri-2-ethylhexanoate.

4. The percutaneous pharmaceutical preparation for external use according to any one of claims 1 to 3, wherein the higher fatty acid ester is one or more selected from the group consisting of isopropyl myristate, isopropyl palmitate, cetyl isooctanoate, diethyl sebacate, diisopropyl adipate and dioctyl adipate.

5. The percutaneous pharmaceutical preparation for external use according to any one of claims 1 to 4, wherein the higher fatty acid ester is one or more selected from the group consisting of higher fatty acid esters which are liquid at ordinary temperatures.

6. The percutaneous pharmaceutical preparation for external use according to any one of claims 1 to 5, wherein the content of the nonsteroidal antiinflammatory analgesic is 0.1 to 10 weight %, the content of the dibenzoylmethane derivative is 0.1 to 20 weight %, and the content of one or more selected from the polyol fatty acid esters, the higher fatty acid esters, and crotamiton is 0.1 to 10 weight %.

7. The percutaneous pharmaceutical preparation for external use according to any one of claims 1 to 6, wherein the dibenzoylmethane derivative is 4-*tert*-butyl-4'-methoxy-dibenzoylmethane, and one or more selected from the polyol fatty acid esters, the higher fatty acid esters, and crotamiton are one or more selected from diisopropyl adipate, propylene glycol dicaprylate, caprylic/capric triglyceride, glyceryl tri-2-ethylhexanoate, and crotamiton.

8. The percutaneous pharmaceutical preparation for external use according to any one of claims 1 to 7, wherein the dosage form of the percutaneous pharmaceutical preparation for external use is a cataplasm or a plaster.

9. The percutaneous pharmaceutical preparation for external use according to any one of claims 1 to 8, wherein the pharmaceutical preparation further comprises a polyisoprene rubber, a natural rubber, an acrylic rubber, a styrene-butadiene-styrene block copolymer, or a styrene-isoprene-styrene block copolymer.

## Patentansprüche

1. Perkutane pharmazeutische Zubereitung zur äußeren Anwendung umfassend die folgenden Bestandteile (A), (B) und (C);
(A) ein nichtsteroidales antiinflammatorisches Analgetikum,
(B) ein Dibenzoylmethanderivat,
(C) einer oder mehr ausgewählt aus Polyolfettsäureester, höhere Fettsäureester und Crotamiton,
wobei das Dibenzoylmethanderivat 4-tert-Butyl-4'-methoxydi-benzoylmethan und das nichtsteroidale antiinflammatorische Analgetikum Ketoprofen oder ein pharmazeutisch akzeptables Salz davon ist.

2. Perkutane pharmazeutische Zubereitung zur äußeren Anwendung nach Anspruch 1, wobei der Gehalt des Dibenzoylmethanderivats 0,1 bis 20 Gew.-% beträgt.

3. Perkutane pharmazeutische Zubereitung zur äußeren Anwen-dung nach einem der Ansprüche 1 oder 2, wobei der Polyolfettsäureester einer oder mehr ist, ausgewählt aus der Gruppe bestehend aus Propylenglykolmonocaprylat, Propylenglykoldicaprylat, Capryl-/Caprin-Triglycerid und Glyceryl-tri-2-ethylhexanoat.

4. Perkutane pharmazeutische Zubereitung zur äußeren Anwendung nach einem der Ansprüche 1 bis 3, wobei der höhere Fettsäureester einer oder mehr ist, ausgewählt aus der Gruppe bestehend aus Isopropylmyristat, Isopropylpalmitat, Cetylisooctanoat, Diethylsebacat, Diisopropyladipat und Dioctyladipat.

5. Perkutane pharmazeutische Zubereitung zur äußeren Anwendung nach einem der Ansprüche 1 bis 4, wobei der höhere Fettsäureester einer oder mehr ist, ausgewählt aus der Gruppe bestehend aus höheren Fettsäureestern, welche bei Normaltemperaturen flüssig sind.

6. Perkutane pharmazeutische Zubereitung zur äußeren Anwendung nach einem der Ansprüche 1 bis 5, wobei der Gehalt an dem nichtsteroidalen antiinflammatorischen Analgetikum 0,1 bis 10 Gew.-% beträgt, der Gehalt an dem Dibenzoylmethanderivat 0,1 bis 20 Gew.-% beträgt und der Gehalt an einem oder mehr ausgewählt aus den Polyolfettsäureestern, den höheren Fettsäureestern und Crotamin 0,1 bis 10 Gew.-% beträgt.

7. Perkutane pharmazeutische Zubereitung zur äußeren Anwendung nach einem der Ansprüche 1 bis 6, wobei das Dibenzoylmethanderivat 4-tert-Butyl-4'-methoxydibenzoylmethan ist und einer oder mehr ausgewählt aus den Polyolfettsäureestern, den höheren Fettsäureestern und Crotamin einer oder mehr ausgewählt aus Diisopropyladipat, Propylenglykoldicaprylat, Capryl-/Caprin-Triglycerid und Glyceryl-tri-2-ethylhexanoat und Crotamiton sind.

8. Perkutane pharmazeutische Zubereitung zur äußeren Anwendung nach einem der Ansprüche 1 bis 7, wobei die Darreichungsform der perkutanen pharmazeutischen Zubereitung zur äußeren Anwendung ein Kataplasma oder ein Pflaster ist.

9. Perkutane pharmazeutische Zubereitung zur äußeren Anwendung nach einem der Ansprüche 1 bis 8, wobei die pharmazeutische Zubereitung weiterhin ein Polyisoprenkautschuk, einen Naturkautschuk, einen Acrylkautschuk, ein Styrol-Butadien-Styrol-Blockcopolymer oder ein Styrol-Isopren-Styrol-Blockcopolymer umfasst.

## Revendications

1. Préparation pharmaceutique percutanée à usage externe comprenant les ingrédients (A), (B) et (C) suivants;
(A) un analgésique anti-inflammatoire non stéroïdien,
(B) un dérivé de dibenzoylméthane,
(C) un ou plusieurs choisis parmi des esters d'acides gras de polyols, esters d'acides gras supérieurs, et crotamiton, dans laquelle le dérivé de dibenzylméthane est du 4-tert-butyl-4'-méthoxydibenzoyl-méthane et l'analgésique anti-inflammatoire non stéroïdien est du kétoprofène ou un sel pharmaceutiquement acceptable de celui-ci.

2. Préparation pharmaceutique percutanée à usage externe selon la revendication 1, dans laquelle la teneur en dérivé de dibenzoylméthane va de 0,1 à 20 % en poids.

3. Préparation pharmaceutique percutanée à usage externe selon l'une des revendications 1 ou 2, dans laquelle l'ester d'acide gras de polyol, un ou plusieurs, est choisi parmi le groupe consistant en monocaprylate de propylène glycol, dicaprylate de propylène glycol, triglycéride caprylique/caprique et tri-2-éthylhexanoate de glycéryle.

4. Préparation pharmaceutique percutanée à usage externe selon l'une des revendications 1 à 3, dans laquelle l'ester d'acide gras supérieur, un ou plusieurs, est choisi parmi le groupe consistant en myristate d'isopropyle, palmitate d'isopropyle, isooctanoate de cétyle, sébaçate de diéthyle, adipate de diisopropyle et adipate de dioctyle.

5. Préparation pharmaceutique percutanée à usage externe selon l'une des revendications 1 à 4, dans laquelle l'ester d'acide gras supérieur, un ou plusieurs, est choisi parmi le groupe consistant en esters d'acides gras supérieurs qui sont liquides à des températures ordinaires.

6. Préparation pharmaceutique percutanée à usage externe selon l'une des revendications 1 à 5, dans laquelle la teneur en analgésique anti-inflammatoire non stéroïdien va de 0,1 à 10 % en poids, la teneur en dérivé de dibenzoylméthane va de 0,1 à 20 % en poids, et la teneur en un ou plusieurs choisis parmi les esters d'acides gras de polyols, les esters d'acides gras supérieurs et le crotamiton va de 0,1 à 10 % en poids.

7. Préparation pharmaceutique percutanée à usage externe selon l'une des revendications 1 à 6, dans laquelle le dérivé de dibenzoylméthane est du 4-tert-butyl-4'-méthoxydibenzoyl-méthane, et un ou plusieurs choisis parmi les esters d'acides gras de polyols, les esters d'acides gras supérieurs et le crotamiton, un ou plusieurs, sont choisis parmi de l'adipate de diisopropyle, dicaprylate de propylène glycol, triglycéride caprylique/caprique, tri-2-éthylhexanoate de glycéryle et crotamiton.

8. Préparation pharmaceutique percutanée à usage externe selon l'une des revendications 1 à 7, dans laquelle la présentation de la préparation pharmaceutique percutanée à usage externe est un cataplasme ou un emplâtre.

9. Préparation pharmaceutique percutanée à usage externe selon l'une des revendications 1 à 8, dans laquelle la préparation pharmaceutique comporte en outre un caoutchouc polyisoprène, un caoutchouc naturel, un caoutchouc acrylique, un copolymère séquencé styrène-butadiène-styrène ou un copolymère séquencé styrène-isoprène-styrène.
